# EUROPÄISCHE PATENTSCHRIFT

(11) **EP 3 669 832 B1**
(45) Veröffentlichungstag und Bekanntmachung des Hinweises auf die Patenterteilung: **13.04.2022**
(21) Anmeldenummer: 19208452.3
(22) Anmeldetag: 12.11.2019
(51) Int. Cl.: A61F 5/01

(54) **SPRUNGGELENKSORTHESE**
ANKLE PROSTHESIS
ORTHÈSE DE CHEVILLE

(30) Priorität: 22.11.2018 DE 102018129472
(43) Veröffentlichungstag der Anmeldung: 24.06.2020
(73) Patentinhaber: Ferd. Hauber GmbH, 72622 Nürtingen (DE)
(72) Erfinder: CARSTENS, Felix, 67433 Neustadt (DE); LAITZSCH, Lukas, 10829 Berlin (DE); HORST, Christian, 73230 Kirchheim u. Teck (DE)
(74) Vertreter: DREISS Patentanwälte PartG mbB

(56) Entgegenhaltungen:
- EP-A1- 0 412 052
- EP-A1- 2 491 895
- DE-A1- 19 826 900
- DE-U1- 20 115 104
- US-A- 4 727 863
- US-A1- 2013 338 558

## Beschreibung

Die vorliegende Erfindung betrifft eine Sprunggelenksorthese gemäß Anspruch 1. Die erfindungsgemäßen Sprunggelenk-Orthesen umfassen einen biegeschlaff ausgebildeten stiefelartigen Grundkörper.

Dieser Grundkörper ist jedoch nach vorne hin offen. Dadurch kann der Grundkörper bzw. die Orthese quasi von der Ferse her in Fußlängsrichtung auf den Fuß aufgeschoben werden. Der Grundkörper ist aus einem flexiblen Textilmaterial gebildet. Ein unteres Ende des Grundkörpers wird durch einen unteren Sohlenabschnitt gebildet.

Der Sohlenabschnitt kann im Bereich der Fußsohle elastisch ausgebildet sein. Der Sohlenabschnitt kann sich damit auf die individuelle Fußbreite einstellen. Es kann weiterhin ein zusätzliches, unelastisches Verbindungselement im Bereich des Sohlenabschnitts vorhanden sein. Dieses Verbindungselement ist vorzugsweise parallel zu dem oben erwähnten elastischen Sohlenabschnitt, kann zweiteilig ausgeführt sein, und dient dazu, den medialen und den lateralen Abschnitt des Grundkörpers einstellbar unelastisch zu verbinden. Die Verbindung kann beispielsweise durch eine Flausch-Klettverbindung hergestellt werden. Oberhalb des hinteren Endes des Sohlenabschnitts weist der Grundkörper einen hinteren Anlageabschnitt für die Wade des Trägers der Orthese auf. Um die Orthese am Fuß beziehungsweise um das Sprunggelenk des Trägers herum anbringen zu können weist die Orthese ein Spannsystem auf. Das Spannsystem ist dazu ausgebildet und angeordnet, die Orthese, insbesondere im Wesentlichen stufenlos, um das Sprunggelenk zu schließen und straff an dieses anzulegen. Die Orthese weist weiter ein mediales Schienenelement sowie ein laterales Schienenelement auf. Das mediale Schienenelement ist biegesteif und insbesondere federnd elastisch ausgebildet. Im angelegten Zustand ist das mediale Schienenelement auf der Fuß- und Unterschenkelinnenseite angeordnet. Das laterale Schienenelement ist ebenso biegesteif und insbesondere federnd elastisch ausgebildet und im angelegten Zustand auf der Fuß- oder Unterschenkelaußenseite angeordnet. Bei der Orthese der erfindungsgemäßen Art sind die beiden Schienenelemente, also das mediale und das laterale Schienenelement, separat voneinander ausgebildet und im angelegten Zustand zueinander beabstandet.

DE 198 26 900 A1, US 2013 / 338 558 A1 und US 4 727 863 A zeigen jeweils Orthesen mit einigen Merkmalen des Anspruchs 1. US 2013/ 338 558 A1 offenbart die Merkmale des Oberbegriffs des Anspruchs 1.

Derartige Orthesen dienen der Stabilisierung des Sprunggelenks. Damit kann zum einen bei bereits geschädigtem Sprunggelenk beispielsweise bei einer Bandverletzung das Sprunggelenk beziehungsweise die Bänder entlastet werden und eine weitere Verschlimmerung der Verletzung verhindert werden zum anderen kann das Tragen einer derartigen Orthese auch einer Verletzung des Sprunggelenks und des verbundenen Bandapparates vorbeugen. Bei den bekannten Sprunggelenksorthesen ist die Abstützwirkung des Sprunggelenks jedoch häufig unzureichend.

Eine stärkere Stabilisierung wird beispielsweise durch insgesamt steifere Orthesen erreicht, die zum Beispiel keine voneinander getrennt ausgebildeten Schienenelemente aufweisen, sondern bei denen ein den Fuß komplett umschließendes Schienenelement den Fuß stärker versteift. Derartige Orthesen sind jedoch vom Tragekomfort her stark eingeschränkt. Ziel der vorliegenden Erfindung ist es daher, eine Sprunggelenksorthese zu schaffen, die das Sprunggelenk ausreichend stabilisiert und dennoch angenehm zu tragen ist und eine gewisse Beweglichkeit des Sprunggelenks erlaubt. Diese Aufgabe wird durch eine Sprunggelenksorthese mit den Merkmalen des Anspruchs 1 gelöst.

Mit anderen Worten, die erfindungsgemäße Sprunggelenksorthese ist nach dem Oberbegriff des Anspruchs 1 ausgebildet und kennzeichnet sich dadurch aus, dass das mediale Schienenelement sich im angelegten Zustand in distaler Richtung gesehen von Höhe des Schienbeinknochens aus wenigstens bis auf Höhe der Mitte, insbesondere bis in das untere (distale) Drittel, insbesondere bis in das untere Sechstel, des Fersenbeins erstreckt und sich mit seinem unteren, unterhalb des Sprunggelenks angeordneten Ende, nach hinten hin bis in das hintere Drittel, insbesondere das hintere Viertel, insbesondere das hintere Sechstel, des Fersenbeins, insbesondere in den Bereich des Fersenbeinhöckers, erstreckt. Das mediale Schienenelement kann sich nach hinten hin (mit seinem distalen Bereich bzw. dem unterhalb der Mitte des Fersenbeins angeordneten Bereichs) auch bis zur Position des Anlageabschnitts für die Wade erstrecken. Dadurch wird erreicht, dass das mediale Schienenelement zum einen an der Wade eine feste Anlage am Schienbein gegenüber dem Schienbeinknochen einnehmen kann und zum anderen sich derart weit nach unten herab erstreckt, dass es das Fersenbein und über das Fersenbein das Sprunggelenk stabilisieren kann. Dabei erstreckt sich das mediale Schienenelement derart weit nach hinten, dass es in den Bereich des Fersenbeinhöckers hineinragt (mit seinem distalen Bereich bzw. dem unterhalb der Mitte des Fersenbeins angeordneten Bereichs), so dass es hier eine direkte stabilisierende Kontaktfläche mit dem Fersenbein hat. Hierdurch kann das Sprunggelenk in vorteilhafter Weise stabilisiert werden. Bei herkömmlichen Orthesen erstreckt sich das mediale Schienenelement nicht derart weit nach hinten, sondern erstreckt sich quasi ohne Erweiterung im unteren Bereich vom Schienbeinknochen kommend abwärts in kontinuierlicher, oder sogar in sich verjüngender, Breite herab. Die erfindungsgemäße Orthese weist jedoch ein mediales Schienenelement auf, das von der Fußinnenseite nach außen gesehen quasi mit dem Fersenbeinhöcker überlappt, so dass es direkt Kraft auf diesen ausüben kann und hierdurch das Sprunggelenk stabilisieren kann. Bei herkömmlichen Sprunggelenksorthesen liegt das mediale Schienenelement eher im Bereich der Fußsohlensehne beziehungsweise im Bereich des Fuß-Längsgewölbes bzw. der Fußwurzelknochen oder dem vorderen Bereich des Fersenbeins an. Hier hat das Schienenelement jedoch wenig knöcherne Anlagefläche so dass eine Kraftübertragung, insbesondere einer exzessiven Supination entgegenwirkend, und eine stabilisierende Wirkung beziehungsweise eine Kraftübertragung nur geringfügig möglich sind.

Die erfindungsgemäße Aufgabe wird auch durch eine Sprunggelenksorthese nach dem Oberbegriff des Anspruchs 1 gelöst, die sich dadurch kennzeichnet, dass das mediale Schienenelement sich im angelegten Zustand in distaler Richtung gesehen von der proximalen Hälfte insbesondere dem proximalen Drittel, insbesondere dem proximalen Viertel, der Orthese aus wenigstens bis zum distalen Sechstel, insbesondere bis zum distalen Achtel, insbesondere im Wesentlichen bis zum distalen Rand der Orthese erstreckt, sich also im Wesentlichen über die Erstreckung der Orthese von oben bis nach unten hin also vom Schienbeinknochen bis hinunter auf Höhe des Fersenbeins (insbesondere bis in das untere (distale) Drittel, insbesondere bis in das untere Sechstel, des Fersenbeins; insbesondere bis in den Bereich des Sohlenabschnitts) erstreckt, wenn die Orthese angelegt ist. Weiter ist das mediale Schienenelement der Orthese derart ausgebildet, dass es nach hinten hin -also nach dorsal- sich bis zum Anlageabschnitt des Grundkörpers für die Wade erstreckt. Hierdurch wird ebenfalls eine Kontaktierung des Fersenbeins beziehungsweise des Fersenbeinhöckers erreicht, wodurch das Sprunggelenk vorteilhaft stabilisiert werden kann.

Das Spannsystem kann zwei in distal-proximaler Richtung übereinander angeordnete hintere Gurte zur Verbindung der Schienenelemente im Bereich der Wade und zur Bildung eines hinteren Anlageabschnitts für die Wade (Sie sind im angelegten Zustand also gegen die Wade gespannt), umfassen. Der obere Gurt kann im oberen Drittel des medialen Schienenelements und/oder des lateralen Schienenelements an diesem angreifen. Der untere der beiden hinteren Gurte kann im mittleren Drittel des medialen Schienenelements und/oder des lateralen Schienenelements an diesem angreifen. Hierdurch kann die Orthese in vorteilhafter Weise straff um die Wade angelegt werden.

Das Spannsystem kann einen vorderen Gurt zur Verbindung der Schienenelemente im Bereich der Unterschenkelvorderseite aufweisen. Gurt kann in den oberen zwei Dritteln des lateralen Schienenelements an zwei zueinander beabstandeten Stellen angreifen. Zudem kann ein Verbindungsmittel zur Befestigung eines Mittelabschnitts des Gurtes an dem medialen Schienenelement vorgesehen sein. Der Gurt kann damit an zwei Punkten am lateralen Schienenelement angreifen und über das Verbindungsmittel in einfacher Weise an dem medialen Schienenelement befestigt werden. Vorzugsweise ist das Verbindungsmittel derart ausgebildet, dass der Gurt beim Befestigen am medialen Schienenelement gespannt werden kann.

Das Spannsystem kann einen vorderen unteren Gurt umfassen, der im unteren Drittel des lateralen Schienenelements an diesem angreift, und ein Verbindungsmittel zur Befestigung des anderen Endes des Gurts an dem medialen Schienenelement aufweist. Das mediale Schienenelement weist zur Aufnahme des Verbindungsmittels eine entsprechende Verbindungsstelle auf. Diese Verbindungsstelle kann an dem nach vorne gerichteten Fortsatz des medialen Schienenelements angeordnet ist. Hierdurch können die beiden Schienenelemente besonders vorteilhaft in straffer und möglichst flächig anliegender Art und Weise gegen den Fuß bzw. den Unterschenkel des Trägers gespannt werden. Durch den vorderen unteren Gurt ist es insbesondere möglich den Fortsatz straff anliegend gegenüber dem Fuß des Trägers zu befestigen.

In einer Fortbildung der Erfindung kann das mediale Schienenelement an seinem unteren Ende einen sich in einer Fußlängsrichtung nach vorne hin (also in Fußlängsrichtung zu den Zehen hin) erstreckenden Fortsatz aufweisen. Dadurch kann die Anlagefläche des Schienenelements weiter erhöht werden und die Stabilisierung verbessert werden. Der sich nach vorne hin erstreckende Fortsatz ist im angelegten Zustand derart ausgebildet und angeordnet, dass er sich in Fußlängsrichtung über den Teil des Grundkörpers, der im angelegten Zustand oberhalb des Sprunggelenks angeordnet ist, hinaus erstreckt. Blickt man also entlang des Beinverlaufs des Trägers in distaler Richtung, so endet der Teil des Grundkörpers, der oberhalb des Sprunggelenks angeordnet ist, entlang der Fußlängsrichtung vor dem Fortsatz. Der Fortsatz erstreckt sich also weiter zu den Zehen des Trägers hin als der Teil des Grundkörpers, der oberhalb des Sprunggelenks angeordnet ist. Der sich nach vorne erstreckende Fortsatz kann ein vorderes Ende des medialen Schienenelements umfassen. Hierdurch bietet die Orthese auch genau dort Abstützung, wo sie benötigt wird nämlich zum einen am Fersenbeinhöcker und zum anderen am Bereich des Fußwurzelknochens bis zum Mittelfußknochen. Hierdurch wird die Auflagefläche des Schienenelementes im Bereich des Fußes vergrößert, wodurch sich der zur Stabilisierung des Sprunggelenks benötigte Druck verringert und das Sprunggelenk somit in vorteilhafterweise gestützt wird.

Bevorzugt ist weiter, wenn das mediale Schienenelement und das laterale Schienenelement an der Orthesenunterseite über ein biegeschlaffes nichtelastisches Verbindungselement miteinander verbunden sind. Dies kann beispielsweise ein flexibler jedoch unelastischer Gurt beziehungsweise ein Band sein. (Beispielsweise kann der Gurt über eine Klettverbindung (Haken- und Ösenverbindung in der Länge variabel sein. Der Gurt kann hierzu doppelt geführt sein.) Hierdurch können die Schienenelemente zwar relativ zueinander bewegt werden und das Anlegen der Orthese ist in einfacher Art und Weise möglich. Im angelegten Zustand ist die Relativposition der beiden Schienenelemente zueinander jedoch festgelegt und die Schienenelemente können gegeneinander gespannt werden.

Die Schienenelemente, also das mediale und das laterale Schienenelement, können beide oder auch eines der Schienenelemente jeweils mit dem Grundkörper unlösbar verbunden sein. Dies kann beispielsweise dadurch realisiert sein, dass die Schienenelemente mit dem Grundkörper vernäht sind. Denkbar sind auch andere Arten der Verbindung der Schienenelemente mit dem Grundkörper. Vorzugsweise sind die Schienenelemente auf der Außenseite des Grundkörper angeordnet, insbesondere unlösbar befestigt.

Vorteilhafterweise ist das Spannsystem derart ausgebildet, das es an dem medialen Schienenelement und/oder an dem lateralen Schienenelement angreift. Die Verspannung der Orthese kann dann durch Verspannung der Schienenelemente erfolgen, so dass der Grundkörper überwiegend durch die Schienenelemente am Fuß angelegt ist und die Einbringung der Spannung über die Schienenelemente selbst erfolgt. Hierzu weist die Orthese vorteilhafterweise wenigstens einen Spanngurt im unteren (distalen) Bereich der Schienenelemente auf und wenigstens einen Spanngurt im oberen (proximalen) der Schienenelemente auf, also in dem Bereich der Schienenelemente, der in proximal-distaler Richtung, auf Höhe des die Wade umschließenden Bereichs des Grundkörpers angeordnet ist. Der Spanngurt im unteren Bereich der Schienenelemente kann an dem Fortsatz des medialen Schienenelements angreifen. Er kann insbesondere mittels eines Clips-Verschlusses oder eines Hakenelements mit dem medialen Schienenelement lösbar verbunden sein. Vorteilhafterweise ist der Spanngurt, der im oberen Bereich angeordnet ist, als v-förmig zulaufendes Spanngurtpaar mit zwei Gurten ausgebildet die an zwei verschiedenen Stellen eines der Schienenelemente angreifen und an ihrem anderen Ende miteinander verbunden sind und vorteilhafterweise über ein Einhaksystem mit der anderen Schiene verbunden werden können. Bevorzugter Weise weist die Orthese im unteren Bereich noch einen Gurt auf, der kreuzartig um den Fuß und die Wade legbar ist. Dieser Gurt ist vorteilhafterweise mit dem Grundkörper verbunden. Dieser zusätzliche Gurt kann beispielsweise über eine Klettverbindung oder ein Haken- und Ösensystem lösbar mit dem Grundkörper verbunden sein.

Der Grundkörper ist vorteilhafterweise derart ausgebildet, dass er im rückseitigen Bereich zwischen dem Sohlenabschnitt und dem Anlageabschnitt des Grundkörpers eine Öffnung zur Aufnahme der Ferse aufweist. Die Öffnung zur Aufnahme der Ferse ist dabei vorzugsweise derart angeordnet und ausgebildet, dass der Sohlenabschnitt sich quasi horizontal erstreckt und in vertikaler Richtung anschließend an den Sohlenabschnitt direkt die Öffnung zur Aufnahme der Ferse folgt und in proximaler Richtung gesehen also aufwärts gesehen auf die Öffnung der Anlageabschnitt des Grundkörpers für die Wade folgt. Mit anderen Worten es kann vorgesehen sein, dass sich im hinteren Bereich (im Bereich der Ferse) vom Sohlenabschnitt kein Material des Grundkörpers in proximaler Richtung nach oben erstreckt, sondern an dieser Stelle direkt die Öffnung angeordnet ist und erst oberhalb der Öffnung wieder Material des Grundkörpers vorhanden ist.

Eine gesonderte Aufgabe der vorliegenden Erfindung ist es, eine Sprunggelenksorthese, die insbesondere nach einer der vorigen Ausführungsformen ausgebildet ist, bereitzustellen, bei der die Biegesteifigkeit wenigstens eines Schienenelements anpassbar ist.

Diese eigenständige Aufgabe wird durch eine Sprunggelenksorthese, die eine eigenständige Erfindung bildet gelöst, wobei die Sprunggelenksorthese insbesondere nach einer der vorigen Ausführungsformen ausgebildet ist, jedoch nicht zwingend nach einer der vorigen Ausführungsformen ausgebildet ist. Bei dieser Sprunggelenksorthese ist ein biegeschlaff ausgebildeter und stiefelartig jedoch nach vorne offener Grundkörper vorgesehen. Dieser Grundkörper ist aus einem flexiblen Textilmaterial gebildet, wie dies auch bei den vorher beschriebenen Ausführungsformen der Fall ist. Der Grundkörper weist über dies ebenfalls den unteren Sohlenabschnitt mit dem über seinem hinteren Ende angeordneten hinteren Anlageabschnitt des Grundkörpers für die Wade auf. Der Grundkörper kann die oben beschriebenen und das Spannsystem betreffenden Aspekte der vorherig beschriebenen Erfindung aufweisen. Die Orthese gemäß dieser eigenständigen Erfindung weist überdies ein Spannsystem auf, das wie bei der vorigen Erfindung auch dazu angeordnet und ausgebildet ist, die Orthese, insbesondere im Wesentlichen stufenlos, um das Sprunggelenk herum zu schließen und straff an dieses anzulegen. Das Spannsystem kann die oben beschriebenen und das Spannsystem betreffenden Aspekte der vorherig beschriebenen Erfindung aufweisen. Die Orthese weist außerdem wenigstens ein aufrüstbares, insbesondere federnd elastisches biegesteifes Schienenelement auf. Die Orthese gemäß dieser eigenständigen Erfindung kennzeichnet sich dadurch, dass die Orthese ein insbesondere federnd elastisches, biegesteifes Einsetzelement umfasst. Das Einsetzelement ist mit dem vorher genannten aufrüstbaren Schienenelement kombinierbar. Zur Kombination mit dem Schienenelement ist dieses Einsetzelement mit dem aufrüstbaren Schienenelement verbindbar, um dessen Biegesteifigkeit zu verstärken beziehungsweise zu erhöhen. Zur Verbindung des Einsetzelements mit dem Schienenelement können wenigstens zwei Verbindungsstellen vorgesehen sein, die insbesondere im oberen und unteren Bereich des Schienenelements angeordnet sind. Dabei können die Verbindungsstellen derart ausgebildet sein, dass das Einsetzelement im verbundenen Zustand in distaler Richtung parallel zu dem Schienenelement erstreckt angeordnet ist, insbesondere flächig an diesem anliegt. Um die gewünschte Stabilisierung, die über eine bloße Addition der Biegesteifigkeiten des Einsetzelements und des Schienenelements hinausgeht, zu erreichen, ist das Einsetzelement im verbundenen Zustand über einen formschlüssigen Hintergriff bzw. formschlüssige Hinterschneidung an den jeweiligen Verbindungsstellen derart mit dem Schienenelement verbunden, dass der Bereich der Verbindungsstellen des Einsetzelements an einer Relativbewegung in proximal-distaler Richtung und/oder in Fußlängsrichtung gegenüber dem Schienenelement gehindert ist. Hierdurch ergibt sich eine Versteifung des aufrüstbaren Schienenelements, die über eine bloße Addition der Biegesteifigkeiten des Einsetzelements und des Schienenelements hinausgeht. Mit anderen Worten die Verbindung der beiden Elemente miteinander wirkt im Hinblick auf ihre Steifigkeit synergistisch.

Das aufrüstbare Schienenelement kann insbesondere das laterale Schienenelement sein.

Vorteilhafterweise ist der formschlüssige Hintergriff an wenigstens einer der Verbindungsstellen derart ausgeführt, dass ein Anlageabschnitt vorhanden ist, der derart ausgebildet ist, dass das Einsetzelement und das Schienenelement in wenigstens drei der vier Quadranten der durch die proximal-distale Richtung sowie die Fußlängsrichtung aufgespannten Ebene flächig derart aneinander anliegen, dass der Bereich der Verbindungsstellen des Einsetzelements an einer Relativbewegung gegenüber dem Schienenelement in proximal-distaler Richtung und in Fußlängsrichtung gehindert ist.

Das Einsetzelement ist vorzugsweise medial vom Schienenelement angeordnet. Das Einsetzelement kann insbesondere zwischen Grundkörper und Schienenelement angeordnet sein. Vorzugsweise kann das Einsetzelement von oben her zwischen Grundkörper und Schienenelement eingeschoben werden. Grundkörper und Schienenelement können insbesondere im Randbereich des Schienenelements miteinander vernäht sein. Es ist jedoch auch eine laterale Anordnung des Einsetzelements ggü. dem Schienenelement denkbar.

Bei der erfindungsgemäßen Orthese mit aufrüstbarem Schienenelement kann weiter vorgesehen sein, dass der formschlüssige Hintergriff wenigstens einer der Verbindungsstellen einen weiteren Anlageabschnitt aufweist, der derart ausgebildet ist, dass das Einsetzelement durch einen entsprechenden formschlüssigen Hintergriff an einer Bewegung in medialer Richtung (wenn das Einsetzelement medial vom Schienenelement angeordnet ist) bzw. in lateraler Richtung (wenn das Einsetzelement lateral vom Schienenelement angeordnet ist) gegenüber dem Schienenelement gehindert ist. Dieser formschlüssige Hintergriff kann durch eine lösbar einrastende Verbindung zwischen Einsetzelement und Schienenelement gebildet sein. Ein derartiger Hintergriff kann beispielsweise durch weitere Anlageflächen gebildet sein, die sich in proximal-distaler Richtung sowie in Fußlängsrichtung in einer Ebene erstreckt, wobei diese Ebene durch die proximal-distale Richtung sowie die Fußlängsrichtung aufgespannt ist. Entsprechend hindert ein Anliegen des Einsetzelements und des Schienenelement in dieser Anlagefläche aneinander eine Bewegung in medial-lateraler Richtung. Hierdurch ist das Einsetzelement quasi in das aufrüstbare Schienenelement einclipsbar.

Im Bereich der Verbindungsstellen kann das Einsetzelement oder das Schienenelement eine Art umlaufende Nut aufweisen, in die das jeweils andere Element einclipsbar ist.

Es kann vorgesehen sein, dass das mediale Schienenelement in den Sohlenabschnitt der Orthese übergeht. Es kann alternativ oder zusätzlich vorgesehen sein, dass das laterale Schienenelement in den Sohlenabschnitt der Orthese übergeht.

Nachfolgend wird eine vorteilhafte Ausführungsform der Erfindung unter Bezugnahme auf die beiliegenden Zeichnungen erläuternd. In der Zeichnung zeigen:
- Figur 1: eine schematische Darstellung eines Fußes sowie der im Fuß und der darüber liegenden Wade befindlichen Knochen sowie eine herkömmliche aus dem Stand der Technik bekannte Schiene einer herkömmlichen Sprunggelenksorthese;
- Figur 2: eine erfindungsgemäße Sprunggelenksorthese schräg von der Fußaußenseite her gesehen;
- Figur 3: die Orthese aus Figur 2 von der Fußinnenseite her gesehen;
- Figur 4: ein Einsetzelement;
- Figur 5: die Orthese aus Figur 2 mit herausgenommenem Einsetzelement; und

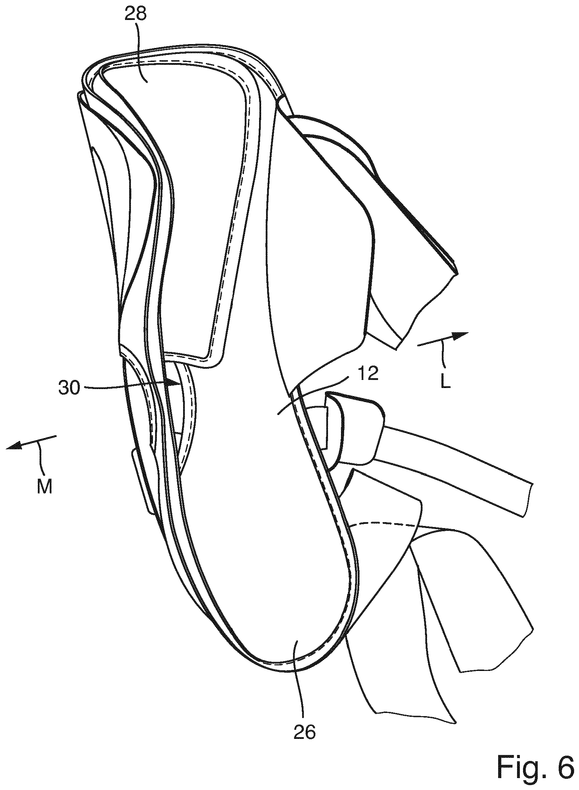

- Figur 6: die Orthese aus den Figuren 2-5 beim Blick von schräg vorne;
- Figur 7: eine weitere erfindungsgemäße Sprunggelenksorthese im aufgeklappten Zustand; und
- Figur 8: eine weitere erfindungsgemäße Sprunggelenksorthese im aufgeklappten Zustand.

In den Figuren tragen entsprechende Elemente und Bereiche gleiche Bezugszeichen. Nicht in allen Figuren sind sämtliche Bezugszeichen widergegeben.

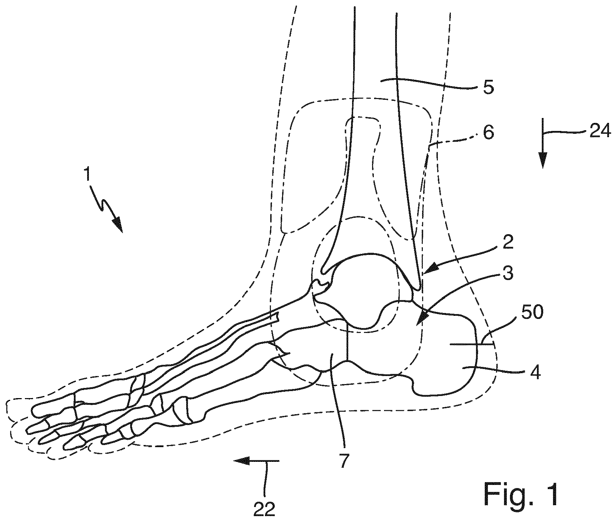

Figur 1 zeigt einen menschlichen Fuß 1 mit einem Sprunggelenk 2. Unterhalb des Sprunggelenks ist ein Fersenbein 3 mit einem Fersenbeinhöcker 4 angeordnet. Oberhalb des Sprunggelenks 2 befindet sich ein Schienbeinknochen 5. Der Wadenbeinknochen ist nicht dargestellt.

Der Fuß 1 aus Figur 1 ist ein linker Fuß und von der Fußaußenseite her blickend dargestellt. Ebenso dargestellt, mittels einer strichpunktierten Linie, ist ein herkömmliches Schienenelement 6 einer herkömmlichen Sprunggelenksorthese. Das Schienenelement 6 der herkömmlichen Orthese erstreckt sich vom Schienbeinknochen 5 hinab bis in den vorderen Bereich des Fersenbeins 3 und überdeckt einen Fußwurzelknochen 7.

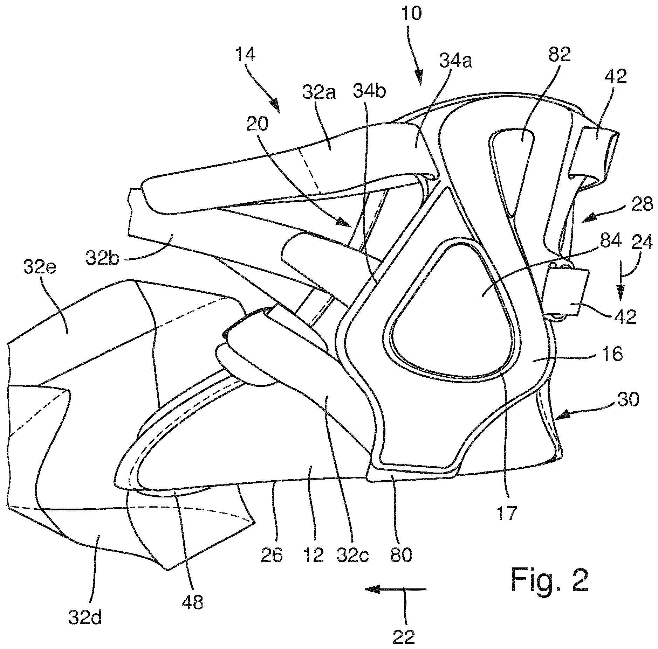

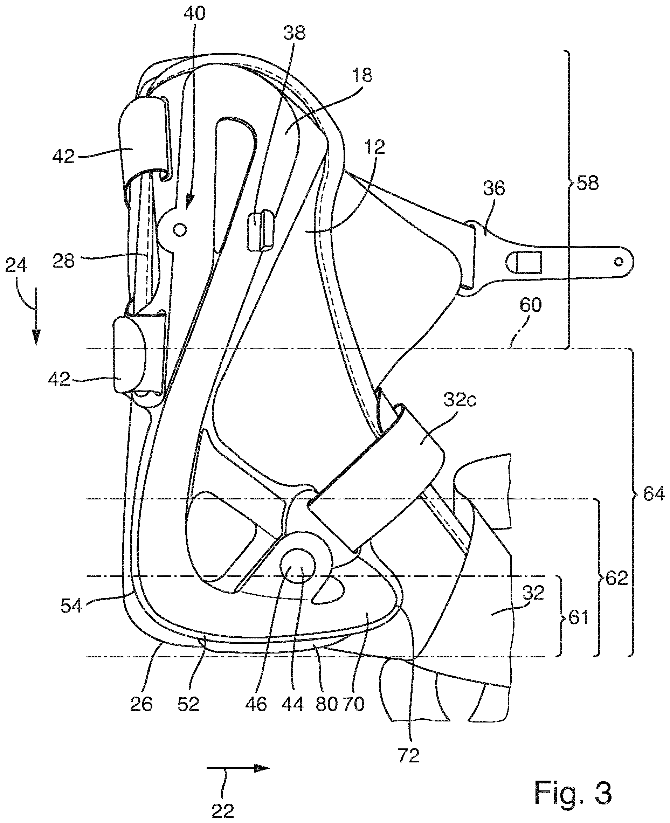

In Figur 2 ist eine erfindungsgemäße Sprunggelenksorthese 10 gezeigt, die zur Verwendung an einem linken Fuß gedacht ist. Die Sprunggelenksorthese 10 weist einen Grundkörper 12 auf und ein Spannsystem 14 sowie ein laterales Schienenelement 16, welches als aufrüstbares Schienenelement 16 ausgebildet ist. Die Orthese aus Figur 2 ist in Figur 3 beim Blick auf die Fußinnenseite - also auf die mediale Seite- gezeigt. In der Darstellung von Figur 3 ist ein mediales Schienenelement 18 sichtbar. Der Grundkörper 12 der Orthese 10 ist aus einem flexiblen Textilmaterial gebildet und in sich biegeschlaff ausgebildet. Von der Form her ist der Grundkörper 12 stiefelartig ausgebildet, wobei er jedoch nach vorne hin eine Öffnung 20 aufweist. Ein Pfeil zeigt nach vorne und in Fußlängsrichtung 22. Entsprechend ist die proximal-distale Richtung 24 durch einen Pfeil gekennzeichnet, der in distale Richtung weist.

Der Grundkörper 12 weist einen unteren Sohlenabschnitt 26 auf, der ein unteres Ende des Grundkörpers 12 bildet. Oberhalb des Sohlenabschnitts 26 bzw. dessen hinteren Endes ist ein hinterer Anlageabschnitt 28 des Grundkörpers 12 angeordnet. Der hintere Anlageabschnitt 28 des Grundkörpers 12, der zur Anlage der Wade des Trägers an dem Grundkörper 12 dient, ist zum unteren Sohlenabschnitt über eine Öffnung 30 beabstandet, die der Aufnahme des Fersenbeins dient. Der Sohlenabschnitt 26 endet im hinteren Bereich flach und geht dann in proximaler Richtung gesehen direkt in die Öffnung 30 zur Aufnahme des Fersenbeins über. Mit anderen Worten im hinteren Bereich erstreckt sich oberhalb des Sohlenabschnitts 26 direkt ein Freibereich bzw. ein materialfreier Bereich, beziehungsweise ein nicht vom Grundkörper bedeckter Bereich. Dieser Bereich geht dann in den Anlageabschnitt 28 zur Anlage der Wade des Trägers über.

Das Spannsystem 14 weist mehrere Gurte 32 a - e auf. Die Gurte 32 a und 32 b sind im oberen Bereich der Schienenelemente 16, 18 angeordnet und greifen beide an separaten Stellen 34a und 34b an dem lateralen Schienenelement 16 an. Sie enden wiederum gemeinsam an einem Spannverschluss 36, der an einer Hakenstelle 38 eingehakt und dann hebelartig in eine Druckknopfaufnahmestellung 40 Stelle 40 eingeschwenkt und dort verclipst werden kann.

Rückseitig sind die Schienenelemente 16 und 18 über Gurte 42 mit klettverschlussbasierter Längenanpassung miteinander verbunden.

Der Spanngurt 32 c ist im unteren Bereich der Schienenelemente 16 und 18 angeordnet und mit dem medialen Schienenelement 18 über eine Clipsverbindung 44 lösbar verbunden. Durch Druck auf einen Druckknopf 46 kann der Spanngurt 32 c von dem medialen Schienenelement 18 gelöst werden. Im vorderen Bereich der Orthese sind die Gurte 32d und 32e angeordnet, diese Gurte sind einstückig miteinander verbunden und über eine Klettverbindung 48 am Grundkörper festgelegt. Sie können schleifenartig bzw. in Form einer sogenannten Achterzügelung um das Sprunggelenk des Trägers gelegt werden, um eine zusätzliche Stabilisierung zu erreichen. Ein Anlegen dieser Gurte reduziert den Zug auf die leicht verletzbaren, bzw. bereits verletzten Bandstrukturen des Sprunggelenkes und wirken dem sogenannten Talusvorschub entgegen.

Das mediale Schienenelement 18 erstreckt sich im angelegten Zustand in distaler Richtung 24 gesehen von Höhe des Schienbeinknochens aus wenigstens bis auf Höhe einer Mitte 50 des Fersenbeins 3. Mit seinem unteren Ende 52, das in distaler Richtung 24 unterhalb des Sprunggelenks 2 angeordnet ist erstreckt es sich weiter nach hinten also entgegen der Fußlängsrichtung 22 gesehen bis in den Bereich des Fersenbeinhöckers 4, den das Schienenelement 6 der herkömmlichen Sprunggelenksorthese unbedeckt lässt.

Insbesondere erstreckt sich das mediale Schienenelement 18 der erfindungsgemäßen Orthese 10 bis zur Position des Anlageabschnitts 28. Mit anderen Worten ein hinteres Ende 54 im unteren Bereich, also in dem Bereich, der unterhalb des Sprunggelenks angeordnet ist, des medialen Schienenelements ist beim Blick in lateraler Richtung, wie er in Figur 3 gezeigt ist in etwa auf gleicher Höhe was die Fußlängsrichtung 22 angeht wie der Anlageabschnitt 28 des Grundkörpers.

In Figur 3 ist eine proximale Hälfte 58 der Orthese oberhalb einer gedachten Mittellinie 60 dargestellt. Entsprechend sind in Figur 3 auch ein distales Achtel 61 beziehungsweise ein distales Viertel 62 beziehungsweise eine distale Hälfte 64 der Orthese 10 dargestellt. Das mediale Schienenelement 18 weist einen Fortsatz 70 auf, der sich nach vorne in Fußlängsrichtung 22 erstreckt. An dem Fortsatz 70 greift der Gurt 32c über eine Clips-Verbindung an. Im angelegten Zustand der Orthese erstreckt sich dieser Fortsatz in Fußlängsrichtung über den Teil des Grundkörpers 12 hinaus, der oberhalb des Sprunggelenks angeordnet ist und der Fortsatz umfasst ein vorderes Ende 72 des medialen Schienenelements 18. Jedenfalls dieses vordere Ende 72 des medialen Schienenelements 18 erstreckt sich also über den Teil des Grundkörpers 12, der im angelegten Zustand oberhalb des Sprunggelenks angeordnet ist, hinaus. Auf der Unterseite der Orthese 10, genauer gesagt unterhalb des Sohlenabschnitts 26, weist die Orthese 10 ein biegeschlaffes beziehungsweise flexibles jedoch unelastisches Verbindungselement 80 zwischen den beiden Schienenelementen 16 und 18 auf. Dieses ist als unelastischer Gurt 80 ausgeführt, um eine Position der Schienenelemente zueinander im angelegten Zustand festzulegen. Das Verbindungselement 80 kann auch zwei Teilgurte umfassen, welche über eine Klettverbindung in ihrer Gesamtlänge einstellbar sind, um die individuelle Fußbreite berücksichtigen zu können.

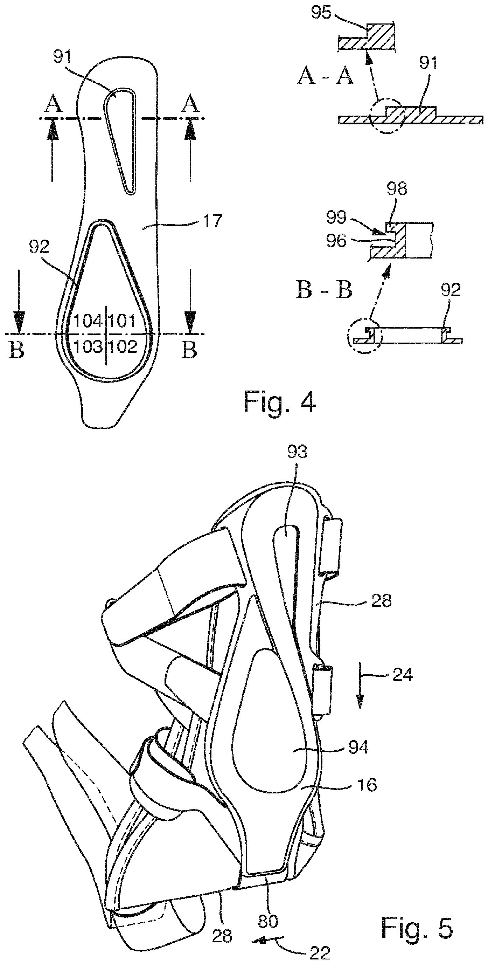

In Figur 4 ist das Einsetzelement 17 des lateralen Schienenelements 16 im von diesem gelösten Zustand gezeigt.

Das federnd elastische und biegesteife Einsetzelement 17 ist mit dem aufrüstbaren biegesteifen, im vorliegenden Fall lateralen, Schienenelement 16 verbindbar.

Für die Verbindung des Einsetzelements 17 mit dem aufrüstbaren Schienenelement 16 sind eine erste Verbindungsstelle 82 und eine zweite Verbindungsstelle 84 vorgesehen. Die erste Verbindungsstelle 82 ist im oberen Bereich des aufrüstbaren Schienenelements 16 angeordnet und die zweite Verbindungsstelle 84 ist im unteren Bereich des aufrüstbaren Schienenelements 16 angeordnet. Im verbundenen Zustand, wie er in Figur 2 gezeigt ist, erstreckt sich das Einsetzelement 17 in distaler Richtung parallel zu dem Schienenelement 16.

Das Einsetzelement 17 ist über einen formschlüssigen Hintergriff an den jeweiligen Verbindungsstellen 82, 84 mit dem Schienenelement 16 verbunden.

Der Bereich der Verbindungsstellen 82, 84 des Einsetzelements 17 ist an einer Relativbewegung in proximal-distaler Richtung 24 und/oder in Fußlängsrichtung 22 gegenüber dem Schienenelement 16 gehindert.

Um diesen Hintergriff zu ermöglichen, weist das Einsetzelement 17 einen in lateralen Richtung L erstreckte Vorsprünge 91 und 92 auf, die in entsprechende Ausnehmungen 93 und 94 des Schienenelements 16 einsetzbar sind. Die Vorsprünge 91 und 92 liegen dann mit ihren in lateral Richtung L erstreckten Seitenwänden 95 und 96 an entsprechenden Seitenwänden der Ausnehmungen 93, 94 an. Hierdurch wird eine Sicherung gegen ein translatorisches Verschieben in proximal-distaler Richtung bzw. in Fußlängsrichtung geschaffen. Um die formschlüssige Verbindung weiter zu verbessern bzw. um eine Verliersicherung des Einsetzelements 17 gegenüber dem Schienenelement 16 zu ermöglichen, weist der Vorsprung 92 zusätzlich einen weiteren Vorsprung 98 auf, der von den Seitenwänden 96 des Vorsprungs 92 abkragt und in der durch die proximal-distale Richtung 24 und die Fußlängsrichtung 22 aufgespannten Ebene erstreckt ist. Hierdurch weist der Vorsprung 92 quasi eine Nut 99 auf, in die ein Bereich des Schienenelements 16 einclipsbar ist. Die Nut 99 ist dabei um den Vorsprung 92 umlaufend ausgebildet. Die Verbindung des Schienenelements 16 mit dem Einsetzelement 17 erhöht zum einen die Biegesteifigkeit und zum anderen auch die Verwindungssteifigkeit.

Zur Verbindung des Einsetzelements 17 mit dem Schienenelement 16 können Vorsprünge statt an dem Einsetzelement 17 auch an dem Schienenelement 16 vorgesehen sein und entsprechende Ausnehmungen an dem Einsetzelement 17. Es kann auch an einer der Verbindungsstellen 82, 84 ein Vorsprung an einem der beiden Teile vorgesehen sein und eine Öffnung bzw. Ausnehmung am anderen Teil und an der anderen Verbindungsstelle die genau umgekehrte Konstellation vorgesehen sein.

Die Vorsprünge dienen zum einen einer Verhinderung einer Verschiebung des Einsetzelements 17 gegenüber dem Schienenelement 16 entlang derer flächigen Erstreckung also im Wesentlichen entlang der proximal-distalen Richtung 24 sowie der Fußlängsrichtung 22. Zum anderen können die Vorsprünge wie oben bereits beschrieben über eine Art Nut bzw. einen weiteren Vorsprung 98 einen Hintergriff aufweisen, der eine Verliersicherung in lateral Richtung L bzw. medialer Richtung M darstellt. Die Verliersicherung hält das Einsetzelement und das Schienenelement also quasi zusammen in flächiger Anlage aneinander.

Die Anlage der Vorsprünge 91 bzw. 92 an ihren Gegenstücken des Schienenelements 16 stellt aneinander anliegende Anlageabschnitte des formschlüssigen Hintergriffs dar. Dabei liegen in dem jeweiligen Anlageabschnitt das Schienenelement 16 und das Einsetzelement 17 in allen vier Quadranten 101, 102, 103 und 104 der durch die proximal-distale Richtung 24 sowie die Fußlängsrichtung 22 aufgespannten Ebene flächig aneinander an.

Im Sinne der Erfindung kann jedoch auch eine Anlage in wenigstens drei der vier Quadranten 101, 102, 103 und 104 sein, wobei die Anlage derart ist, dass der Bereich der Verbindungsstellen 82, 84 des Einsetzelements 17 an einer Relativbewegung gegenüber dem Schienenelement 16 in proximal-distaler Richtung 24 und in Fußlängsrichtung 22 gehindert ist.

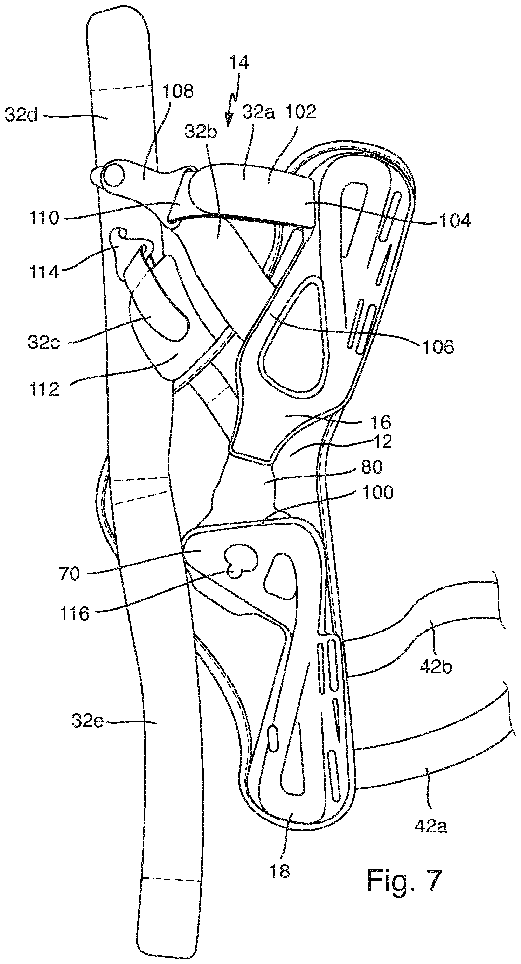

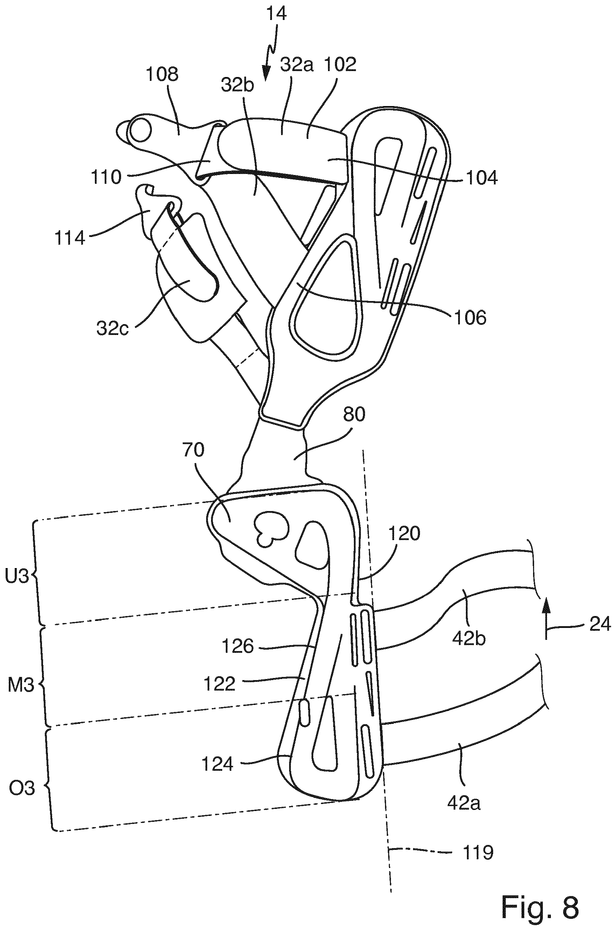

In den Figuren 7 und 8 sind weitere erfindungsgemäße Sprunggelenksorthesen dargestellt.

Die in Figur 7 dargestellte Orthese weist Schienenelemente 16, 18 auf, die im Wesentlichen entsprechend denen der vorangehend beschriebenen Ausführungsform ausgebildet sind. Das Gleiche gilt für die Ausführungsform der Figur 8.

Die in Figur 7 gezeigte Ausführungsform weist einen Grundkörper 12 auf, der sowohl nach vorne wie auch nach hinten hin offen ist. Der Grundkörper 12 verbindet also die Schienenelemente 16, 18 lediglich im unteren Bereich.

Bei beiden in den Figuren 7 und 8 gezeigten Orthesen erstreckt sich das mediale Schienenelement 18 im vorgesehenen angelegten Zustand in distaler Richtung 24 gesehen von oberhalb des Sprunggelenks 2 aus abwärts wenigstens bis zur Höhe der Mitte 50 des Fersenbeins 3 (insbesondere bis in das untere (distale) Drittel, insbesondere bis in das untere Sechstel; vorliegend geht das mediale Schienenelement 18 in den Sohlenabschnitt der Orthese über.). Mit seinem unteren, unterhalb des Sprunggelenks 2 angeordneten Ende 100 erstreckt sich das mediale Schienenelement 18 nach hinten hin bis in das hintere Drittel, insbesondere hintere Viertel, insbesondere hintere Sechstel, des Fersenbeins 3.

Im vorgesehenen angelegten Zustand erstreckt sich das mediale Schienenelement 18 bis in den Bereich des Fersenbeinhöckers 4. Des Weiteren erstreckt sich das mediale Schienenelement 18 bei den beiden Ausführungsformen bis zur Position des Anlageabschnitts 28 für die Wade. Der Anlageabschnitt 28 wird im angelegten Zustand der Orthese 10 durch die beiden Gurte 42a und 42b gebildet. Bei den Orthesen 10 von Figur 7 und Figur 8 ist im hinteren Bereich zwischen den Gurten 42a und 42b und der Wade des Trägers kein Teil des Grundkörpers 12 angeordnet. Zur Polsterung kann hier jedoch ein Teil des Grundkörpers 12 angeordnet sein oder ein separates Polstermittel. Falls im hinteren Bereich zwischen den Gurten 42a und 42b und der Wade des Trägers ein Teil des Grundkörpers 12 angeordnet ist, so bildet dieser ebenfalls einen Anlageabschnitt 28.

Die beiden vorderen Gurte 32a und 32b dienen zum Verbinden der Schienenelemente 16, 18 im Bereich der Unterschenkelvorderseite. Genau betrachtet sind die beiden vorderen Gurte 32a und 32b als ein Gurt 102 ausgebildet, der zur Verbindung der Schienenelemente 16, 18 im Bereich der Unterschenkelvorderseite dient. Dabei greift dieser Gurt 102 in den oberen zwei Dritteln des lateralen Schienenelements 16 an zwei zueinander beabstandeten Stellen 104 und 106 an. Der Gurt 102 umfasst ein Verbindungsmittel 108, das entsprechend dem Spannverschluss 36, wie er in Figur 3 illustriert ist, ausgebildet ist. Über dieses Verbindungsmittel, ist ein Mittelabschnitt 110 des Gurts 102 an dem medialen Schienenelement 18 befestigbar bzw. festlegbar. Der Mittelabschnitt 110 kann allerdings durch die Schlaufe an dem Verbindungsmittel 108 gleiten, so dass die beiden Schenkel 32a und 32b sich automatisch an die individuellen Gegebenheiten anpassen können.

Der Gurt 32c bildet einen vorderen unteren Gurt 112, der im unteren Drittel des lateralen Schienenelements 16 an diesem angreift. Der Gurt 112 weist in der vorliegenden Form ein Verbindungsmittel 114 eines hakenartigen Verschlusses auf. Dieses Verbindungsmittel 114 dient zur Befestigung des losen Endes des Gurtes an dem medialen Schienenelement 18. Zur Befestigung weist das mediale Schienenelement 18 eine entsprechende Verbindungsstelle 116 auf. Die Verbindungsstelle 116 ist dabei an dem Fortsatz 70 des medialen Schienenelements 18 angeordnet.

Fußsohlenseitig sind die beiden Schienenelemente 16, 18 im Fall der Orthese von Figur 7 über einen Sohlenabschnitt bildenden Teil des Grundkörpers 12 sowie über das Verbindungselement 80 verbunden. Bei der Orthese von Figur 8 sind die beiden Schienenelemente 16, 18 über das Verbindungselement 80 verbunden. Das Verbindungselement 80 erstreckt sich zwischen den beiden Schienenelementen 16 und 18 und ist vorliegend längenveränderbar ausgebildet. Vorliegend ist das Verbindungselement 80 als unelastischer Gurt 80 ausgeführt. Die relative Position der Schienenelemente 16, 18 zueinander im angelegten Zustand ist über den Gurt 80 festlegbar. Das Verbindungselement 80 kann auch zwei Teilgurte umfassen, welche über eine Klettverbindung in ihrer Gesamtlänge einstellbar sind, um die individuelle Fußbreite berücksichtigen zu können.

Das mediale Schienenelement 18 ist im Wesentlichen derart ausgebildet, dass es in seinem unteren Drittel U3 im Wesentlichen so weit nach hinten erstreckt ist, wie im darüber liegenden Teil des Schienenelements 18, was durch die Linie 119 illustriert ist. Der darüberliegende Teil des Schienenelements 18 wird dabei durch das obere Drittel O3 und das mittlere Drittel M3 gebildet. Lediglich in dem Bereich, in dem der untere hintere Gurt 42b an dem medialen Schienenelement 18 angreift, steht die hintere Kante des medialen Schienenelements 18 leicht weiter vor als im unteren Bereich. Dies bildet quasi eine Öse zur Befestigung des hinteren unteren Gurts 42b. Im Wesentlichen erstreckt sich die hintere Kante 120 des medialen Schienenelements 18 jedoch geradlinig in distal-proximaler Richtung 24. Im unteren Drittel U3 erstreckt sich das mediale Schienenelement weiter nach vorne als im darüber liegenden Teil des Schienenelements 18. Auf seiner Vorderseite bzw. entlang seiner Vorderkante weist das mediale Schienenelement in seinem unteren Drittel U3 den nach vorne erstreckten Fortsatz 70 auf, wobei der Fortsatz 70 den am weitesten nach vorne erstreckten Teil des medialen Schienenelements 18 bildet. Das mediale Schienenelement weist entlang seiner Vorderkante 122 von oben her zunächst einen vorstehenden Bereich 124 auf, auf den dann in distaler Richtung ein zurückversetzter Bereich 126 folgt.

## Patentansprüche

1. Sprunggelenk-Orthese (10) zum Stabilisieren eines menschlichen Sprunggelenks (2), umfassend:
einen biegeschlaff ausgebildeten stiefelartigen jedoch nach vorne offenen Grundkörper (12), der aus einem flexiblen Textilmaterial gebildet ist, mit einem unteren Sohlenabschnitt (26) oberhalb dessen hinteren Endes ein hinterer Anlageabschnitt (28) des Grundkörpers (12) für die Wade angeordnet ist,
ein Spannsystem (14), das dazu ausgebildet und angeordnet ist, die Orthese (10), insbesondere im Wesentlichen stufenlos, um das Sprunggelenk (2) zu schließen und straff an dieses anzulegen,
wobei die Orthese (10) ein, insbesondere federnd elastisches, biegesteifes mediales Schienenelement (18), das im angelegten Zustand auf der Fuß- und Unterschenkelinnenseite angeordnet ist, und ein,
insbesondere federnd elastisches, biegesteifes laterales Schienenelement (16), das im angelegten Zustand auf der Fuß- und Unterschenkelaußenseite angeordnet ist, umfasst, wobei die Schienenelemente (16, 18) separat voneinander ausgebildet sind und im angelegten Zustand zueinander beabstandet sind,
wobei das mediale Schienenelement (18) sich im angelegten Zustand in distaler Richtung (24) gesehen von oberhalb des Sprunggelenks (2) aus wenigstens bis auf Höhe der Mitte (50) des Fersenbeins (3) erstreckt,
**dadurch gekennzeichnet, dass** das mediale Schienenelement (18) sich im angelegten Zustand
mit seinem unteren, unterhalb des Sprunggelenks (2) angeordneten Ende, nach hinten hin bis in das hintere Drittel, insbesondere das hintere Viertel, insbesondere das hintere Sechstel, des Fersenbeins (3), insbesondere in den Bereich des Fersenbeinhöckers (4), erstreckt insbesondere bis zur Position des Anlageabschnitts (28) für die Wade erstreckt.

2. Sprunggelenk-Orthese (10) nach Anspruch 1, **dadurch gekennzeichnet, dass** das mediale Schienenelement (18) an seinem unteren Ende einen sich in einer Fußlängsrichtung (22) nach vorne erstreckenden Fortsatz (70) aufweist, der in Fußlängsrichtung (22) über den Teil des Grundkörpers (12), der im angelegten Zustand oberhalb des Sprunggelenks (2) angeordnet ist, hinaus erstreckt, und/oder wobei der Fortsatz (70) ein vorderes Ende des medialen Schienenelements (18) umfasst.

3. Sprunggelenk-Orthese (10) nach einem der vorigen Ansprüche, **dadurch gekennzeichnet, dass** das mediale Schienenelement (18) und das laterale Schienenelement (16) an der Orthesenunterseite über ein biegeschlaffes nicht elastisches Verbindungselement (80) miteinander verbunden sind.

4. Sprunggelenk-Orthese (10) nach einem der vorigen Ansprüche, **dadurch gekennzeichnet, dass** das mediale Schienenelement (18) und/oder das laterale Schienenelement (16) mit dem Grundkörper (12) unlösbar verbunden sind.

5. Sprunggelenk-Orthese (10) nach einem der vorigen Ansprüche, **dadurch gekennzeichnet, dass** das Spannsystem an dem medialen Schienenelement (18) und/oder an dem lateralen Schienenelement (16) angreift.

6. Sprunggelenk-Orthese (10) nach einem der vorigen Ansprüche, **dadurch gekennzeichnet, dass** der Grundkörper (12) im rückseitigen Bereich zwischen dem Sohlenabschnitt (26) und dem Anlageabschnitt (28) des Grundkörpers (12) eine Öffnung (30) zur Aufnahme der Ferse aufweist.

## Claims

1. Ankle joint orthosis (10) for stabilizing a human ankle joint (2), comprising:
a non-rigid, boot-shaped, open-fronted base body (12) made from a flexible textile material, with a lower sole segment (26), above the rear end of which is a rear insert segment (28) of the base body (12) for the calf, a bracing system (14) designed and positioned to (mostly gradually) close the orthosis (10) around the ankle joint (2) and to hold it firmly against it,
whereby the orthosis (10) is a rigid, medial splint element (18), in particular a flexible, elastic element, that when worn is positioned on the inside of the foot and lower leg, and a flexible, elastic lateral splint element (16) that when worn is positioned on the outside of the foot and lower leg, whereby the splint elements (16, 18) are separate components and, when worn, are spaced apart from each other,
whereby the medial splint element (18), when worn and viewed distally (24) from above the ankle joint (2), reaches up to the middle (50) of the calcaneus (3), **characterized in that** the medial splint element (18), when worn, extends with its lower end beneath the ankle joint (2) back to the rear third, in particular the rear quarter, in particular the rear sixth, of the calcaneus (3), in particular up to the calcaneal tuberosity (4), in particular until the position of the insert segment (28) for the calf.

2. Ankle joint orthosis (10) as per claim 1, **characterized in that** the medial splint element (18) comprises on its lower end a forward extension (70) in the direction of the length of the foot (22), positioned in the length of the foot (22) above the part of the base body (12) that, when worn, is above the ankle joint (2), and/or whereby the extension (70) comprises a front end of the medial splint element (18).

3. Ankle joint orthosis (10) as per one of the preceding claims, **characterized in that** the medial splint element (18) and the lateral splint element (16) on the underside of the orthosis are connected via a non-rigid, non-elastic connecting element (80).

4. Ankle joint orthosis (10) as per one of the preceding claims, **characterized in that** the medial splint element (18) and/or lateral splint element (16) are irreversibly connected to the base body (12).

5. Ankle joint orthosis (10) as per one of the preceding claims, **characterized in that** the bracing system grasps the medial splint element (18) and/or the lateral splint element (16).

6. Ankle joint orthosis (10) as per one of the preceding claims, **characterized in that** the base body (12) comprises an opening (30) for receiving the calf in the rear portion between the sole segment (26) and the insert segment (28) of the base body (12).

## Revendications

1. Orthèse d'articulation de cheville (10) pour stabiliser une articulation de cheville (2) humaine, comprenant :
un corps de base (12) réalisé de manière souple et flexible du type botte mais ouvert vers l'avant, qui est formé d'un matériau textile flexible, avec une partie semelle inférieure (26) au-dessus de l'extrémité arrière de laquelle est disposée une partie d'appui (28) arrière du corps de base (12) pour le mollet,
un système de serrage (14), qui est réalisé et disposé pour fermer l'orthèse (10), en particulier sensiblement de manière continue, afin de fermer l'articulation de cheville (2) et de la placer fermement contre celui-ci,
dans laquelle l'orthèse (10) comprend un élément attelle médian (18) rigide en flexion, en particulier élastique à ressort, qui est disposé dans l'état placé sur la face intérieure de pied et de jambe, et un élément attelle latéral (16) rigide en flexion, en particulier élastique à ressort, qui est disposé dans l'état placé sur la face extérieure de pied et de jambe, dans laquelle les éléments attelles (16, 18) sont réalisés séparément l'un de l'autre et sont espacés l'un de l'autre dans l'état placé,
dans laquelle
l'élément attelle médian (18) s'étend dans l'état placé dans la direction radiale (24) vu depuis le dessus de l'articulation de cheville (2) à partir d'au moins jusqu'au niveau du centre (50) du calcanéum (3),
**caractérisée en ce que** l'élément attelle médian (18) s'étend dans l'état placé avec son extrémité inférieure, disposée au-dessous de l'articulation de cheville (2), vers l'arrière jusque dans le tiers arrière, en particulier le quart arrière, en particulier le sixième arrière, du calcanéum (3), en particulier dans la zone de la bosse de calcanéum (4), en particulier jusqu'à la position de la partie d'appui (28) pour le mollet.

2. Orthèse d'articulation de cheville (10) selon la revendication 1, **caractérisée en ce que** l'élément attelle médian (18) présente à son extrémité inférieure un prolongement (70) s'étendant vers l'avant dans une direction longitudinale de pied (22), qui s'étend dans la direction longitudinale de pied (22) au-delà de la partie du corps de base (12), qui est disposée dans l'état placé au-dessus de l'articulation de cheville (2), et/ou dans laquelle le prolongement (70) comprend une extrémité avant de l'élément attelle médian (18).

3. Orthèse d'articulation de cheville (10) selon l'une quelconque des revendications précédentes, **caractérisée en ce que** l'élément attelle médian (18) et l'élément attelle latéral (16) sur la face inférieure d'orthèse sont reliés l'un à l'autre par l'intermédiaire d'un élément de liaison (80) non élastique souple et pliable.

4. Orthèse d'articulation de cheville (10) selon l'une quelconque des revendications précédentes, **caractérisée en ce que** l'élément attelle médian (18) et/ou l'élément attelle latéral (16) sont reliés au corps de base (12) de manière non détachable.

5. Orthèse d'articulation de cheville (10) selon l'une quelconque des revendications précédentes, **caractérisée en ce que** le système de serrage agit sur l'élément attelle médian (18) et/ou sur l'élément attelle latéral (16).

6. Orthèse d'articulation de cheville (10) selon l'une quelconque des revendications précédentes, **caractérisée en ce que** le corps de base (12) présente dans la zone arrière entre la partie semelle (26) et la partie d'appui (28) du corps de base (12) une ouverture (30) pour la réception du talon.
